(19) Europäisches Patentamt
European Patent Office
Office européen des brevets

(11) EP 4 755 948 A1

(12) **EUROPEAN PATENT APPLICATION**
published in accordance with Art. 153(4) EPC

(43) Date of publication:
**10.06.2026 Bulletin 2026/24**

(21) Application number: **24848373.7**

(22) Date of filing: **02.08.2024**

(51) International Patent Classification (IPC):
***C08G 73/18*** *(2006.01)* ***C07D 235/04*** *(2006.01)*

(52) Cooperative Patent Classification (CPC):
**B01D 53/02; B01J 20/26; B01J 20/30; C07D 235/04; C08G 73/18; C09K 9/02; G01N 21/64;** Y02E 60/50

(86) International application number:
**PCT/CN2024/109412**

(87) International publication number:
**WO 2025/026419 (06.02.2025 Gazette 2025/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **02.08.2023 CN 202310967288**

(71) Applicants:
- **China Petroleum & Chemical Corporation**
  **Beijing 100728 (CN)**
- **Sinopec (Beijing) Research Institute of Chemical Industry Co., Ltd.**
  **Beijing 100013 (CN)**

(72) Inventors:
- **LI, Jing**
  **Beijing 100013 (CN)**
- **WU, Jianing**
  **Beijing 100013 (CN)**
- **ZHANG, Longgui**
  **Beijing 100013 (CN)**
- **WANG, Ailian**
  **Beijing 100013 (CN)**
- **NIU, Xinmiao**
  **Beijing 100013 (CN)**
- **JI, Wenxi**
  **Beijing 100013 (CN)**
- **ZHANG, Taoyi**
  **Beijing 100013 (CN)**
- **CHEN, Jing**
  **Beijing 100013 (CN)**
- **MAN, Yi**
  **Beijing 100013 (CN)**

(74) Representative: **Hoffmann Eitle Patent- und Rechtsanwälte PartmbB**
**Arabellastraße 30**
**81925 München (DE)**

(54) **POLYBENZIMIDAZOLE, PREPARATION METHOD THEREFOR, AND USE THEREOF**

(57) The present invention provides a novel class of polybenzimidazole polymers that exhibit an excellent gas adsorption separation property while also have a mechanochromic property. The polybenzimidazole polymer of the present invention can be used for adsorption separation of carbon dioxide-containing gases.

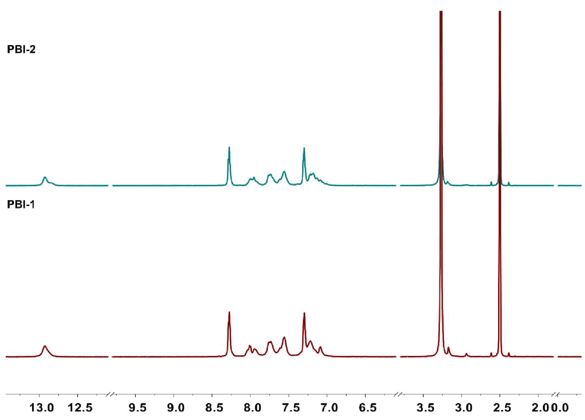

Fig. 1

EP 4 755 948 A1

## Description

Technical Field

The present invention relates to the field of polymer materials, more particularly to a polybenzimidazole comprising tetraphenylethylene group, a method for separating gases by adsorption using the same and the use thereof for gas adsorption separation.

Backgrounds

Polybenzimidazole (PBI) refers to a class of heterocyclic polymers containing imidazole ring in repeating units, and has excellent thermal stability, mechanical stability and chemical stability.

Due to excellent processability, polymers are usually processed into membrane products such as nanofiltration membranes, hollow fiber membranes and the like for separation and purification of gases. Although excellent in separation performance, polymer membrane products generally do not have an adsorption effect. The present invention provides novel polybenzimidazole polymers which exhibit a good adsorption effect on gas and exhibit different adsorption selectivities on different gases.

Summary of Invention

The invention aims to provide a novel polybenzimidazole polymer having a gas adsorption separation property. In addition, the polybenzimidazole polymer of the present application has a mechanochromic property.

The first aspect of the invention provides a polybenzimidazole, the polybenzimidazole comprises structural unit A, and the structural unit A has a structure represented by formula (1);

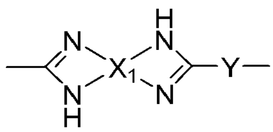
formula (1)

wherein, the $X_1$ group is a linking group provided by an aromatic hydrocarbon compound containing 1-10 benzene rings; and the Y group is a tetraphenylethylene-based linking group; wherein, the $X_1$ group and the Y group are each independently not substituted or substituted by at least one substituent selected from the group consisting of hydroxyl, amino, cyano, halogen, $C_1$-$C_4$ alkyl, and $C_1$-$C_4$ haloalkyl.

In some embodiments, the polybenzimidazole comprises structural unit A and structural unit C, comprises structural unit A and structural unit D, or comprises structural unit A, structural unit B, structural unit C, and structural unit D; wherein the structural unit A has a structure represented by formula (1), the structural unit B has a structure represented by formula (2), the structural unit C has a structure represented by formula (1-1), and the structural unit D has a structure represented by formula (2-2):

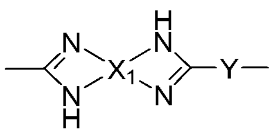
formula (1)
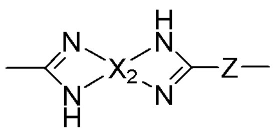
formula (2)

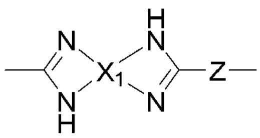
formula (1-1)
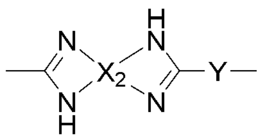
formula (2-2)

wherein, the $X_1$ and $X_2$ groups are each independently a linking group provided by an aromatic hydrocarbon compound containing 1-10 benzene rings; the Y group is a tetraphenylethylene-based linking group; and the Z group is a linking group provided by at least one compound selected from the group consisting of an aromatic hydrocarbon containing 1-10 benzene rings, a heterocyclic aromatic hydrocarbon containing 1-10 heterocyclic rings, an aromatic hydrocarbon containing 1-3 heterocyclic rings and 1-8 benzene rings, a $C_4$-$C_{10}$ cycloalkane, a $C_1$-$C_8$ paraffinic hydrocarbon and a $C_2$-$C_8$ olefin; wherein, the $X_1$, $X_2$, Y and Z groups are each independently not substituted or substituted by at least one

substituent selected from the group consisting of hydroxyl, amino, cyano, halogen, $C_1$-$C_4$ alkyl, and $C_1$-$C_4$ haloalkyl.

In some embodiments, the polybenzimidazole comprises structural unit A and optionally structural unit B, the structural unit A having a structure represented by formula (1) and the structural unit B having a structure represented by formula (2);

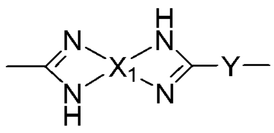
formula (1)
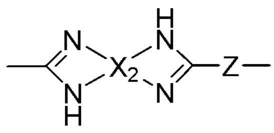
formula (2)

wherein, the $X_1$ and $X_2$ groups are each independently a linking group provided by an aromatic hydrocarbon compound containing 1-10 benzene rings; the Y group is a tetraphenylethylene-based linking group; and the Z group is a linking group provided by at least one compound selected from the group consisting of an aromatic hydrocarbon containing 1-10 benzene rings, a heterocyclic aromatic hydrocarbon containing 1-10 heterocyclic rings, an aromatic hydrocarbon containing 1-3 heterocyclic rings and 1-8 benzene rings, a $C_4$-$C_{10}$ cycloalkane, a $C_1$-$C_8$ paraffinic hydrocarbon and a $C_2$-$C_8$ olefin, and Z is different from Y when the structural unit B exists; wherein, the $X_1$, $X_2$, Y and Z groups are each independently not substituted or substituted by at least one substituent selected from the group consisting of hydroxyl, amino, cyano, halogen, $C_1$-$C_4$ alkyl, and $C_1$-$C_4$ haloalkyl.

The present invention also provides a method for preparing the polybenzimidazole of the invention, which comprises reacting at least one compound having a structure represented by formula (6) with HOOC-Y-COOH and optionally HOOC-Z-COOH;

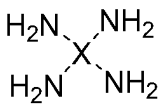
formula (6)

wherein the X group in formula (6) is the same as the $X_1$ and/or $X_2$ groups defined in the first aspect and the Y and Z groups in HOOC-Y-COOH and optionally HOOC-Z-COOH are respectively the same as the Y and Z groups defined in the first aspect.

The invention also provides a method for separating $CO_2$ gas by adsorption, wherein the polybenzimidazole of the invention is used as adsorbent.

The invention also provides an use of the polybenzimidazole of the invention as adsorbent for gas adsorption separation.

Description of Drawings

FIG. 1 is the hydrogen nuclear magnetic resonance spectra of the polymer (PBI-1) of Example 1 and the polymer (PBI-2) of Example 2.
FIG. 2 is the free volume probability density function graph of PBI-4.
FIG. 3 is the fluorescence spectra of PBI-2 before and after grinding.
FIG. 4 is the pictures of real products of a non-inventive polybenzimidazole polymer (left) and the polymer of Example 4 (PBI-4, right).

Detailed Description

The first aspect of present invention a provides polybenzimidazole, the polybenzimidazole comprises structural unit A, and the structural unit A has a structure represented by formula (1);

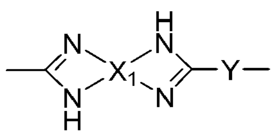
formula (1)

wherein, the $X_1$ group is a linking group provided by an aromatic hydrocarbon compound containing 1-10 benzene rings;

and the Y group is a tetraphenylethylene-based linking group; wherein, the $X_1$ group and the Y group are each independently not substituted or substituted by at least one substituent selected from the group consisting of hydroxyl, amino, cyano, halogen, $C_1$-$C_4$ alkyl, and $C_1$-$C_4$ haloalkyl. In some embodiments, the polybenzimidazole consists of the structural unit A.

In some embodiments, the polybenzimidazole comprises structural unit A and structural unit C, comprises structural unit A and structural unit D, or comprises structural unit A, structural unit B, structural unit C, and structural unit D; wherein the structural unit A has a structure represented by formula (1), the structural unit B has a structure represented by formula (2), the structural unit C has a structure represented by formula (1-1), and the structural unit D has a structure represented by formula (2-2):

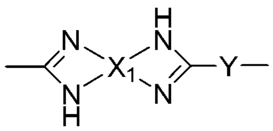 formula (1) 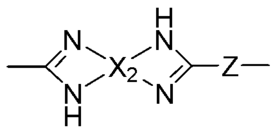 formula (2)

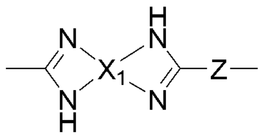 formula (1-1) 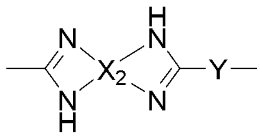 formula (2-2)

wherein, the $X_1$ and $X_2$ groups are each independently a linking group provided by an aromatic hydrocarbon compound containing 1-10 benzene rings; the Y group is a tetraphenylethylene-based linking group; and the Z group is a linking group provided by at least one compound selected from the group consisting of an aromatic hydrocarbon containing 1-10 benzene rings, a heterocyclic aromatic hydrocarbon containing 1-10 heterocyclic rings, an aromatic hydrocarbon containing 1-3 heterocyclic rings and 1-8 benzene rings, a $C_4$-$C_{10}$ cycloalkane, a $C_1$-$C_8$ paraffinic hydrocarbon and a $C_2$-$C_8$ olefin; wherein, the $X_1$, $X_2$, Y and Z groups are each independently not substituted or substituted by at least one substituent selected from the group consisting of hydroxyl, amino, cyano, halogen, $C_1$-$C_4$ alkyl, and $C_1$-$C_4$ haloalkyl. In some embodiments, the polybenzimidazole consists of structural unit A and structural unit C. In some embodiments, the polybenzimidazole consists of structural unit A and structural unit D. In some embodiments, the polybenzimidazole consists of structural unit A, structural unit B, structural unit C, and structural unit D.

In some embodiments, the polybenzimidazole comprises structural unit A and optionally structural unit B, the structural unit A having a structure represented by formula (1) and the structural unit B having a structure represented by formula (2);

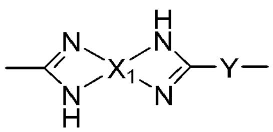 formula (1) 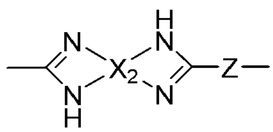 formula (2)

wherein, the $X_1$ and $X_2$ groups are each independently a linking group provided by an aromatic hydrocarbon compound containing 1-10 benzene rings; the Y group is a tetraphenylethylene-based linking group; the Z group is a linking group provided by at least one compound selected from the group consisting of an aromatic hydrocarbon containing 1-10 benzene rings, a heterocyclic aromatic hydrocarbon containing 1-10 heterocyclic rings, an aromatic hydrocarbon containing 1-3 heterocyclic rings and 1-8 benzene rings, a $C_4$-$C_{10}$ cycloalkane, a $C_1$-$C_8$ paraffinic hydrocarbon and a $C_2$-$C_8$ olefin, and Z is different from Y when the structural unit B exists; wherein the $X_1$, $X_2$, Y and Z groups are each independently not substituted or substituted by at least one substituent selected from the group consisting of hydroxyl, amino, cyano, halogen, $C_1$-$C_4$ alkyl, and $C_1$-$C_4$ haloalkyl.

In the present invention, the linking ways of the $X_1$ and $X_2$ groups in formula (1) and formula (2) are known to those skilled in the art. For example, when the $X_1$ and $X_2$ groups are each independently a linking group provided by benzene, then the structural formulas of formula (1) and formula (2) can be respectively

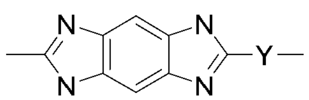 and 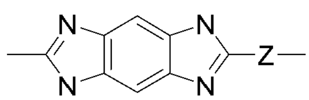 ;

that is, the benzene ring and imidazole rings share two pairs of carbon atoms.

In the present invention, $C_4$-$C_{10}$ cycloalkane can be, for example, cyclobutane, cyclopentane, cyclohexane, cycloheptane, cyclooctane, cyclononane or cyclodecane.

In the present invention, $C_1$-$C_8$ paraffinic hydrocarbon can be, for example, methane, ethane, n-propane, isopropane, butane, pentane, hexane, heptane, octane. In some embodiments, $C_1$-$C_8$ paraffinic hydrocarbon can be a $C_1$-$C_8$ paraffinic hydrocarbon substituted by one or more halogen atoms, which can be selected from the group consisting of fluorine atom, chlorine atom, and bromine atom.

In the present invention, $C_2$-$C_8$ olefin can be, for example, ethylene, propylene, butene, pentene, hexene, heptene, or octene.

In the present invention, halogen can be selected from, for example, fluorine, chlorine and bromine.

In the present invention, $C_1$-$C_4$ alkyl can be, for example, methyl, ethyl, n-propyl, isopropyl, n-butyl, sec-butyl, isobutyl, or tert-butyl.

In the present invention, $C_1$-$C_4$ haloalkyl can be, for example, halomethyl, haloethyl, halo-n-propyl, halo-isopropyl, halo-n-butyl, halo-sec-butyl, halo-isobutyl, halo-tert-butyl; the halogen atom(s) in the $C_1$-$C_4$ haloalkyl can be, for example, fluorine atom, chlorine atom and bromine atom.

According to the present invention, preferably, in some embodiments, the $X_1$ and $X_2$ groups are each independently a linking group provided by at least one compound selected from the group consisting of benzene, biphenyl, terphenyl, a bridged-benzenes, an iptycene, and a fused ring aromatic hydrocarbon containing 1-10 benzene rings; preferably, the $X_1$ and $X_2$ groups are each independently a linking group provided by at least one compound selected from the group consisting of benzene, biphenyl, a bridged-benzenes, an iptycene, and naphthalene; more preferably, the bridged-benzenes has a structure represented by formula (3), formula (4) or formula (5), wherein $R_1$, $R_3$, $R_4$ are each independently selected from the group consisting of oxygen atom, sulfur atom, carbonyl, sulfonyl, methylene, and halogen-substituted methylene; and $R_2$ is selected from the group consisting of heteroatom-containing aromatic rings;

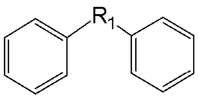 formula (3) 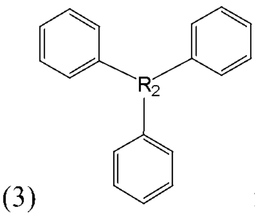 formula (4) 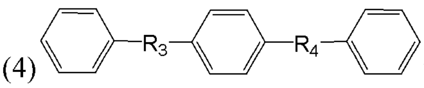 formula (5).

In the present invention, the heteroatom-containing aromatic ring can be, for example, pyridine or pyrimidine.

In the present invention, the linking positions (indicated by "dotted line") of the structures represented by formulas (3), (4) and (5) can be as follows:

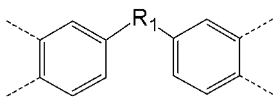, 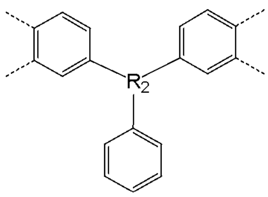 and 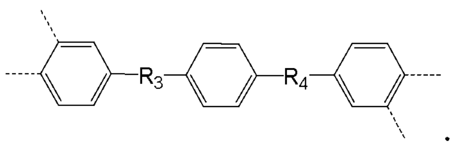.

In the present invention, the linking positions include, but not limited to, above linking positions; for example, any two adjacent linking positions on the same benzene ring are possible.

According to the present invention, preferably, the $X_1$ and $X_2$ groups are each independently selected from the following linking groups:

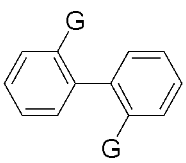 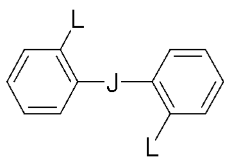 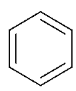 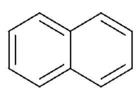

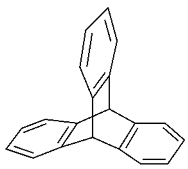 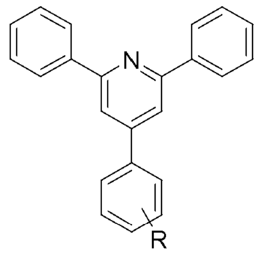 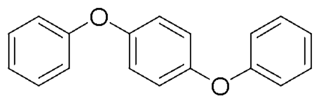 ;

wherein G is selected from the group consisting of hydrogen atom, methyl and trifluoromethoxy; J is selected from the group consisting of oxygen atom, sulfur atom, carbonyl, sulfonyl, methylene and methyl- or trifluoromethyl-substituted methylene; L is selected from the group consisting of bromine atom, phenyl and trifluoromethyl-substituted phenyl; and R is selected from the group consisting of hydrogen atom, carboxyl, hydroxyl and trifluoromethyl.

In present invention, in some embodiments, the linking positions in the $X_1$ and $X_2$ groups can include, but not limited to, the linking positions shown below:

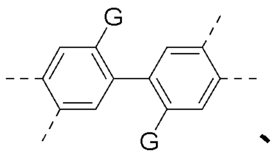 、 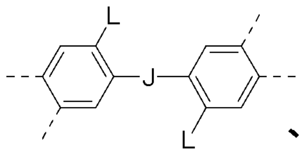 、 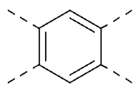 、 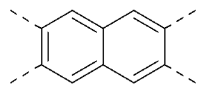 、

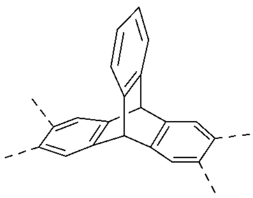 、 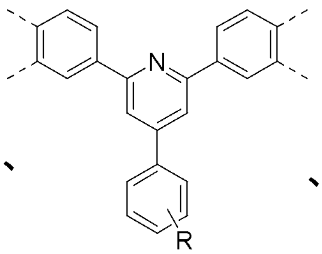 、 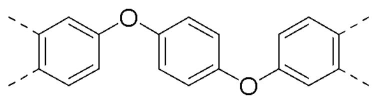 .

According to the present invention, in some embodiments, the molar content ratio of the $X_2$ group to $X_1$ group can be 0-9999, preferably 0-99; for example, the ratio can be 0, 0.001, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 10, 20, 30, 40, 50, 60, 70, 80, 90, 99, 100, or a range formed by any two of above values. In the present invention, in the absence of specific description, the content ratio of the $X_2$ structural unit or group to $X_1$ structural unit or group in polybenzimidazole is calculated from the feeding amounts.

According to the invention, preferably, the Y group is derived from at least one of the following compounds:

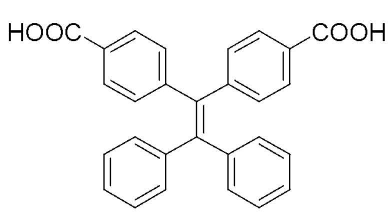 and 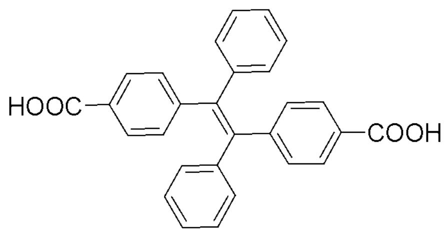 .

According to the present invention, preferably, in some embodiments, the Z group is a linking group provided by at least one compound selected from the group consisting of benzene, a 5-6 membered nitrogen-containing heterocyclic compound, an aromatic hydrocarbon compound containing 2-7 benzene rings, an aromatic hydrocarbon compound containing 1-2 nitrogen-containing heterocyclic rings and 1-4 benzene rings, a $C_4$-$C_{10}$ cycloalkane, a $C_1$-$C_8$ paraffinic hydrocarbon, and a $C_2$-$C_8$ olefin.

According to the invention, preferably, the Z group is a linking group selected from:

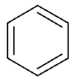 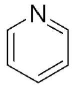 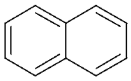 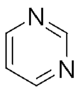 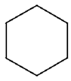 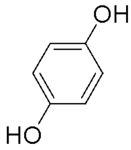 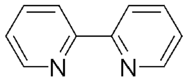 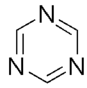

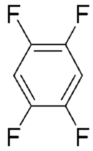 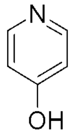 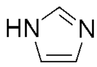 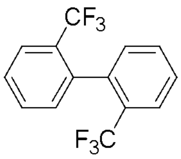 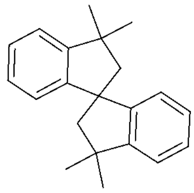 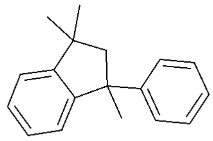

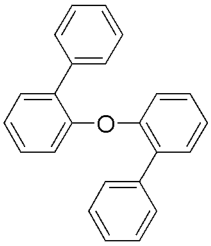 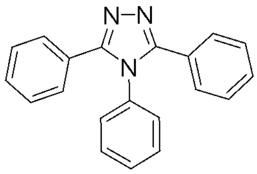 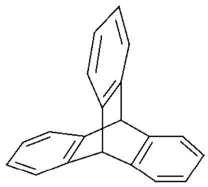 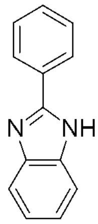 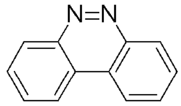

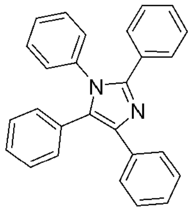 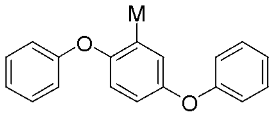 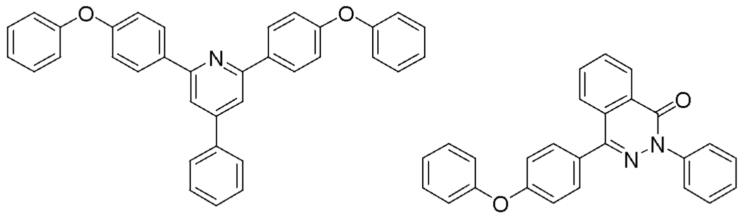

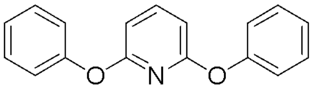 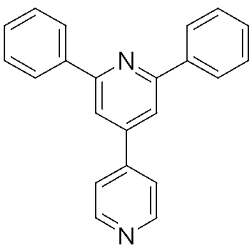 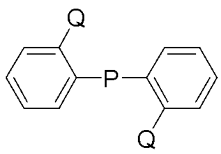

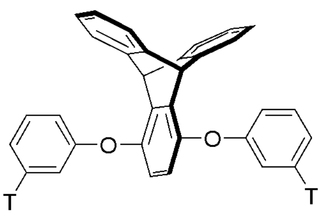 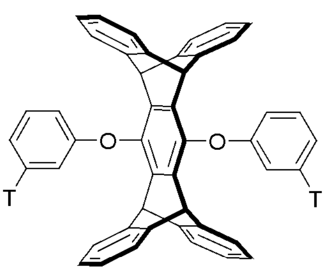 ,

$C_1$-$C_8$ paraffinic hydrocarbon group;

wherein M is selected from the group consisting of hydrogen atom, cyano, methyl, phenyl and methyl- or trifluoromethyl-substituted phenyl; P is selected from the group consisting of oxygen atom, sulfur atom, carbonyl, sulfonyl, cyclobutyl, halogen-substituted cyclobutyl, methylene and methyl- or trifluoromethyl-substituted methylene; Q is selected from the group consisting of bromine atom, phenyl and trifluoromethyl-substituted phenyl; and T is selected from the group consisting of hydrogen atom and methyl.

In the present invention, there are no particular limits to the linking positions of the Z group, and the linking

positions can be any position on the linking group. For example, when the Z group contains a cyclic structure, the linking position can be a carbon atom on the cyclic structure; when the Z group is a $C_1$-$C_8$ paraffinic hydrocarbon group, the linking position can be any carbon atom in the paraffinic hydrocarbon group. In the present invention, the linking positions of the Z group can include, but not limited to, the linking positions shown below:

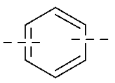
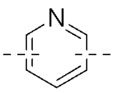
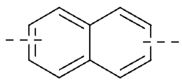
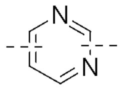
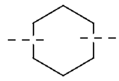
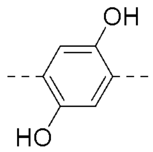
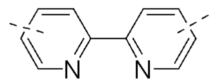
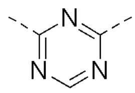

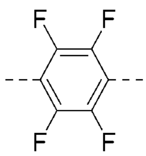
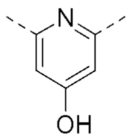
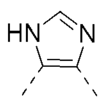
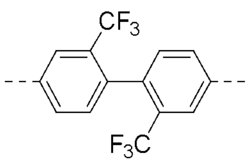
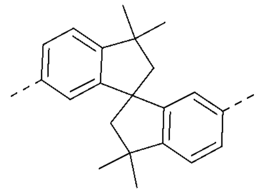
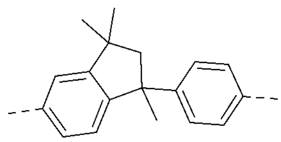

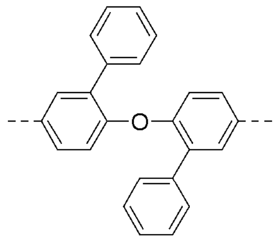
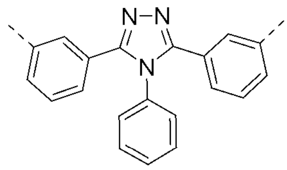
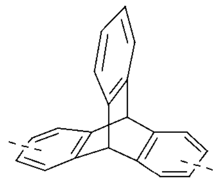
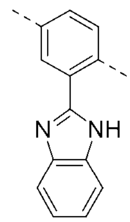
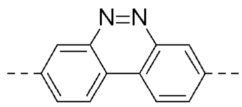

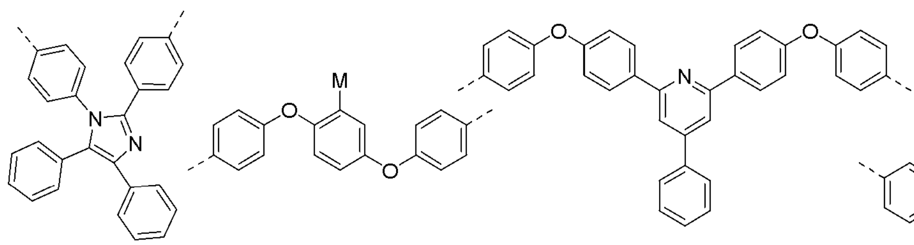
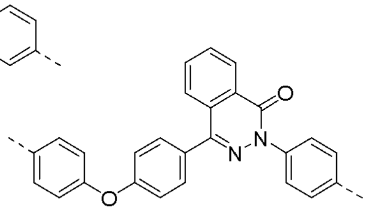

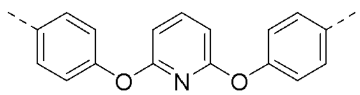
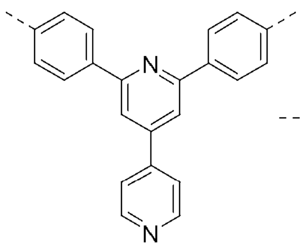
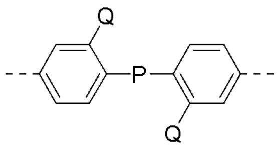
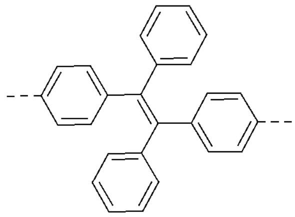

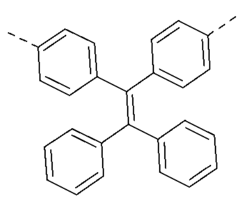
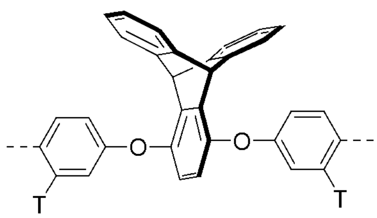
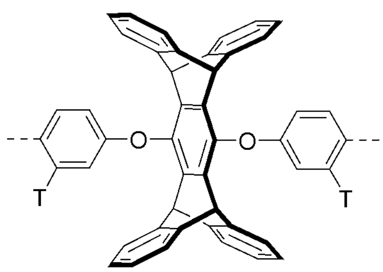
.

According to the invention, the molar content ratio of the Z group to the Y group can be 0-9999, more preferably 0-99; for example, the ratio can be 0, 0.001, 0.01, 0.02, 0.03, 0.04, 0.05, 0.06, 0.07, 0.08, 0.09, 0.1, 0.2, 0.3, 0.4, 0.5, 0.6,

0.7, 0.8, 0.9, 1, 2, 3, 4, 5, 10, 20, 30, 40, 50, 60, 70, 80, 90, 99, 100, or a range formed by any two of above values. In the present invention, in the absence of specific description, the content ratio of Z structural unit or group to Y structural unit or group in polybenzimidazole is calculated from the feeding amounts.

In the present invention, the free volume of the polybenzimidazole can be 80-120Å$^3$, preferably 85-110Å$^3$; and the pore diameter of the polybenzimidazole can be 2.5-3.5Å, preferably 2.6-3.2Å.

In the present invention, the polybenzimidazole can have an intrinsic viscosity of 0.45 to 3.0 dL g$^{-1}$; for example, the intrinsic viscosity can be 0.45 dL g$^{-1}$, 0.5 dL g$^{-1}$, 0.6 dL g$^{-1}$, 0.7 dL g$^{-1}$, 0.8 dL g$^{-1}$, 0.9 dL g$^{-1}$, 1 dL g$^{-1}$, 1.1 dL g$^{-1}$, 1.2 dL g$^{-1}$, 1.3 dL g$^{-1}$, 1.4 dL g$^{-1}$, 1.5 dL g$^{-1}$, 1.6 dL g$^{-1}$, 1.7 dL g$^{-1}$, 1.8 dL g$^{-1}$, 1.9 dL g$^{-1}$, 2.0 dL g$^{-1}$, 2.1 dL g$^{-1}$, 2.2 dL g$^{-1}$, 2.3 dL g$^{-1}$, 2.4 dL g$^{-1}$, 2.5 dL g$^{-1}$, 2.6 dL g$^{-1}$, 2.7 dL g$^{-1}$, 2.8 dL g$^{-1}$, 2.9 dL g$^{-1}$, 3.0 dL g$^{-1}$, and a range formed by any two of above values.

The preparation of polybenzimidazoles is known in the art. The polybenzimidazole of the present invention can be prepared by using various preparation methods known in the art.

In another aspect, the present invention also provides a method for preparing the polybenzimidazole of the first aspect, the method comprises reacting at least one compound having the structure represented by formula (6) with HOOC-Y-COOH and optionally HOOC-Z-COOH;

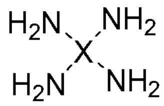 formula (6)

wherein the X group in formula (6) is the same as the $X_1$ and/or $X_2$ groups in above formula (1) and formula (2), and the Y group and Z group in HOOC-Y-COOH and optionally HOOC-Z-COOH are respectively the same as the Y group and Z group defined in above formula (1) and formula (2), and thus are not described here again.

According to the preparing method of the present invention, preferably, in some embodiments, the compound having the structure represented by formula (6) is at least one selected from the following compounds:

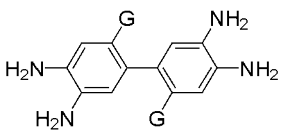 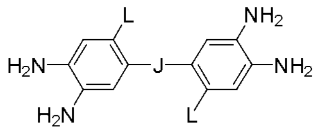 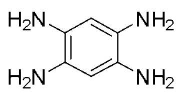 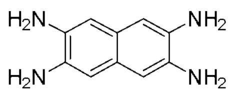

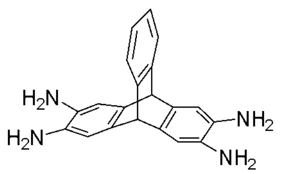 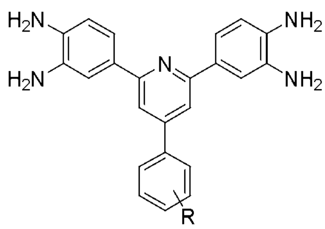 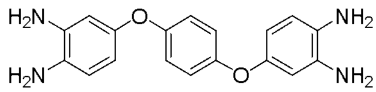 ;

wherein G is selected from the group consisting of hydrogen atom, methyl and trifluoromethoxy, i.e. -H, $-CH_3$, $-OCF_3$; J is selected from the group consisting of oxygen atom, sulfur atom, carbonyl, sulfonyl, methylene, and methyl- or trifluoromethyl-substituted -O-, -S-, methylene, such as

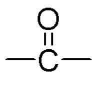 , 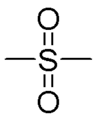 , 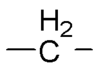 , 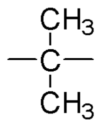 , 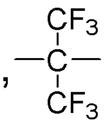 ;

L is selected from the group consisting of bromine atom, phenyl and trifluoromethyl-substituted phenyl, such as

—Br, 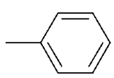 , 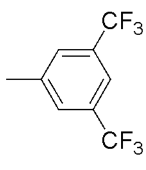 ;

and
R is selected from the group consisting of hydrogen atom, carboxyl, hydroxyl and trifluoromethyl, i.e., -H, -COOH, -OH, $-CF_3$.

According to the preparing method of the present invention, preferably, in some embodiments, the reaction is performed in a solvent, more preferably, the solvent is polyphosphoric acid or a mixed solution of methanesulfonic acid/phosphorus pentoxide, further preferably a mixed solution of methanesulfonic acid/phosphorus pentoxide and the mass of phosphorus pentoxide is 2-10% of the mass of methanesulfonic acid. The concentration of the mixed monomers in the reaction system can be 3 wt% to 20 wt%.

According to the preparing method of the present invention, preferably, in some embodiments, the ratio of the molar amount of the compound having the structure represented by formula (6) to the total molar amount of HOOC-Y-COOH and optionally HOOC-Z-COOH is 1:0.6-2, preferably 1:0.8-1.2.

According to the preparing method of the present invention, preferably, in some embodiments, the reaction time can be 1 to 9 hours, preferably 3 to 5 hours. The reaction temperature can be 60 to 230 °C, preferably 60 to 170 °C, more preferably 100 °C to 150 °C. The reaction pressure can be atmospheric pressure.

According to the preparing method of the present invention, preferably, HOOC-Y-COOH is selected from at least one of the following compounds:

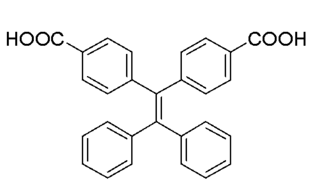 and 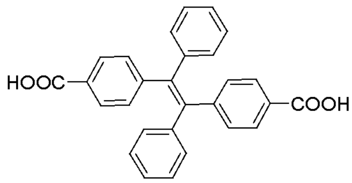 .

According to the preparing method of the present invention, preferably, in some embodiments, HOOC-Z-COOH is selected from at least one of the following compounds:

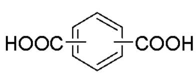 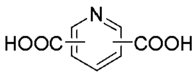 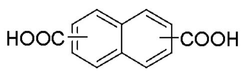 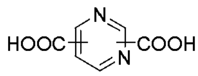 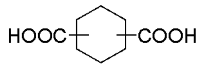

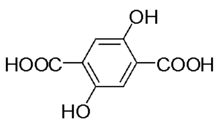 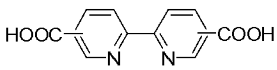 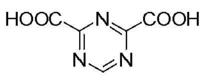 $HOOC-(CF_2)_n-COOH$ $HOOC-(CH_2)_n-COOH$

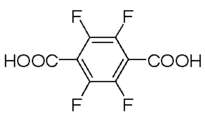 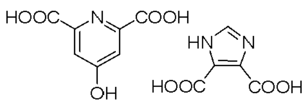 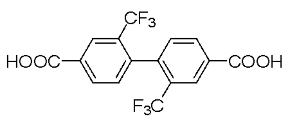 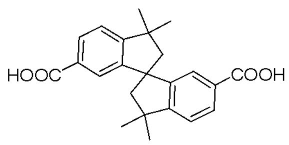

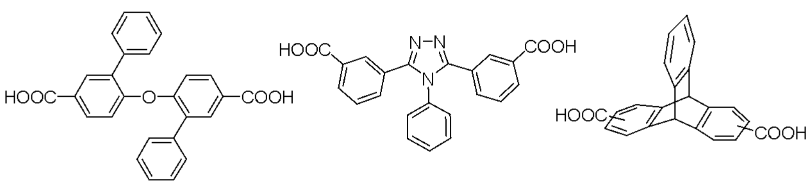 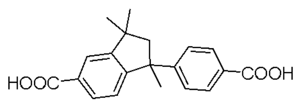

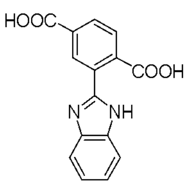 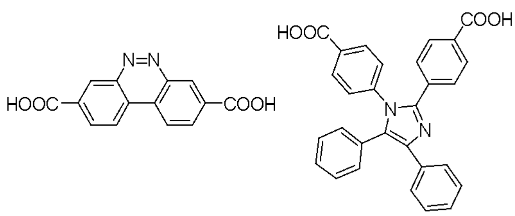 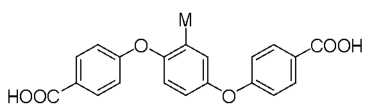

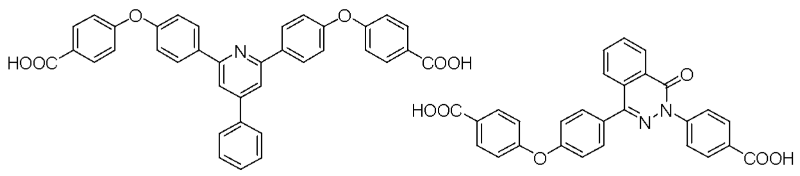
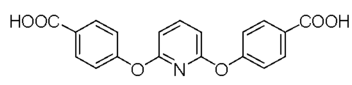

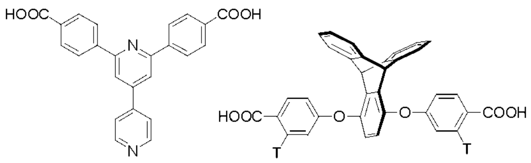
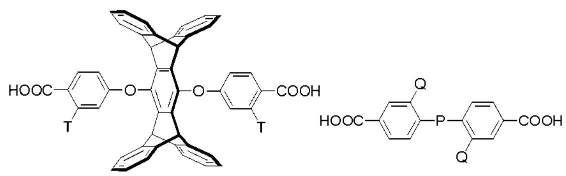

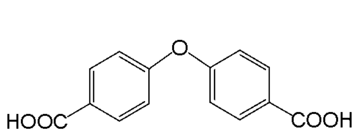
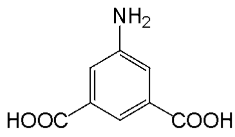;

wherein n is 1-8; M, P, Q, T each represents a substituent, which are the same as those described in the first aspect and thus are not described here again.

According to the preparing method of the present invention, preferably, in some embodiments, the ratio of the molar amount of the compound having the structure represented by formula (6) to the total molar amount of HOOC-Y-COOH and optionally HOOC-Z-COOH is 1:0.6-2; for example, 1:0.6, 1:0.7, 1:0.8, 1:0.9, 1:1, 1:1.1, 1:1.2, 1:1.3, 1:1.4, 1:1.5, 1:1.6, and a range formed by any two of above values; preferably 1:0.8-1.2; wherein the molar content ratio of the $X_2$ group to the $X_1$ group can be 0-9999, preferably 0-99, and for example, the ratio can be 0, 0.001, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 10, 20, 30, 40, 50, 60, 70, 80, 90, 99, 100, and a range formed by any two of above values; the molar content ratio of Z group to Y group can be 0-9999, preferably 0-99, and for example, the ratio can be 0, 0.001, 0.01, 0.02, 0.03, 0.04, 0.05, 0.06, 0.07, 0.08, 0.09, 0.1, 0.2, 0.3, 0.4, 0.5, 0.6, 0.7, 0.8, 0.9, 1, 2, 3, 4, 5, 10, 20, 30, 40, 50, 60, 70, 80, 90, 99, 100, and a range formed by any two of above values.

In some embodiments, the polybenzimidazole of the present invention can have one of the structures shown below:

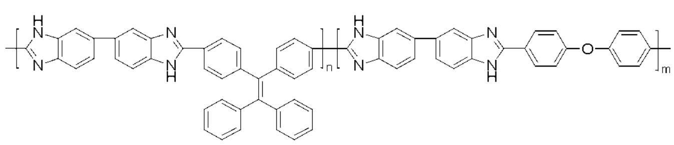,
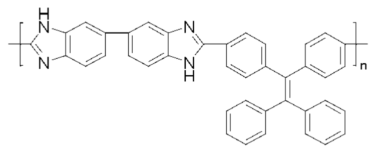

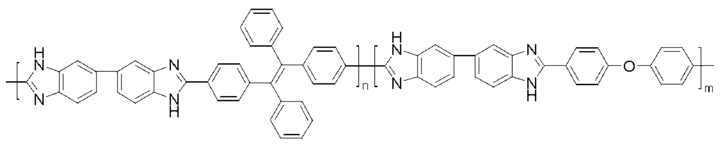,
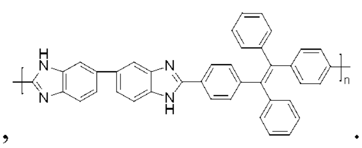.

In the structures shown above, with respect to copolymers, the structures merely show that the polymer chains comprise the shown repeating structural units or that the polymer chains consist of the shown repeating structural units, and do not show the distributions of the shown repeating structural units in the polymer chains.

The present invention also provides the use of the above polybenzimidazole polymers in the aspect of gas adsorption separation.

In one aspect, the present invention provides a method for separating $CO_2$ gas by adsorption, wherein the polybenzimidazole according to the first aspect of the invention is used as adsorbent.

It has been surprisingly found in the present invention that the polybenzimidazole of the present invention has a high adsorption capacity for $CO_2$ and a low adsorption capacity for $N_2$ and $O_2$, thereby providing a high carbon dioxide/nitrogen separation coefficient and a high carbon dioxide/oxygen separation coefficient, making it useful for separating $CO_2$ from a gas selected from the group consisting of $N_2$, $O_2$ or a mixture thereof.

The present invention has no limits to practical separation operations, but generally includes contacting the

polybenzimidazole of the present invention with a gas mixture comprising $CO_2$. When the polybenzimidazole of the present invention adsorbs a sufficient amount of $CO_2$, the polybenzimidazole can be removed and subjected to desorption and regeneration. The regenerated polybenzimidazole can be contacted again with a gas mixture comprising $CO_2$ to adsorb $CO_2$.

The invention also provides the use of a polybenzimidazole according to the first aspect of the invention as adsorbent for gas adsorption separation. Since the polybenzimidazole of present invention has a high adsorption capacity for $CO_2$ and has a low adsorption capacity for $N_2$ and $O_2$, the polybenzimidazole of present invention can be used as adsorbent to separate $CO_2$ from a gas selected from the group consisting of $N_2$, $O_2$ or a mixture thereof.

The present invention provides a polybenzimidazole polymer which has an advantageous gas adsorption separation property and which has an excellent thermal stability, making it useful in gas adsorption separation applications in some high temperature situations.

In addition, the polybenzimidazole polymer of the present invention has a mechanochromic property. A mechanochromic material means when the material is subjected to an external force stimulus, such as a stimulus of grinding, stretching, compressing or other stimuli, the color property, luminescence property or the like of the material are rendered changed. The polybenzimidazole polymer of the invention has an obviously shifted fluorescence emission peak after the stimulus of external force, so that the polybenzimidazole polymer is a mechanochromic material.

In some embodiments, the polybenzimidazole of the present invention can be used for flow rate monitoring of a carbon dioxide-containing gas. For example, the polybenzimidazole of the present invention can be coated on the inner surface of a microchannel and then arranged at the end of a gas channel where the flow rate or emission of carbon dioxide is to be monitored. When carbon dioxide slightly leaks, the carbon dioxide can be absorbed by virtue of its adsorption property; when the leakage amount of the carbon dioxide increases, the material is subjected to a force due to the impact of flow rate to cause a change in fluorescence emission peak signal which is captured by a detector and then an alarm is given.

Polybenzimidazoles which do not contain tetraphenylethylene groups are generally red to brown in color, while polybenzimidazoles which contain tetraphenylethylene groups are generally yellow in color. The diversity of the color of polybenzimidazoles is increased due to the incorporation of tetraphenylethylene groups, and the application scenarios of polybenzimidazoles can be expanded. FIG. 4 shows pictures of real products of a non-inventive polybenzimidazole polymer (left; prepared by using 3,3',4,4'-tetraaminobiphenyl and 4,4'-diphenylether dicarboxylic acid in a molar ratio of 1:1.05 in a way similar to the method of the Examples of present application) and the polymer of Example 4 (PBI-4, right).

In order that the invention can be more readily understood, the invention will be described in detail with reference to the Examples, which are for illustration only and are not limited to the scope of application of the invention. It should be noted that reasonable modifications made by persons skilled in the art inspired by this patent are within the scope of protection of this patent.

In the following Examples,

the yield of the polymerization product is calculated by the following equation:

$$\text{yield} = \text{actual amount of polymer} \div \text{theoretical amount of polymer} \times 100\%;$$

wherein the theoretical amount of polymer = the molar amount of the compound represented by formula (6) $\times$ the molar mass of the repeating unit(s) in the polymer structure.

3,3',4,4'-tetraaminobiphenyl: Sigma-Aldrich; purity 98%;

4,4'-diphenyl ether dicarboxylic acid: J&K Scientific; purity 98%;

4,4'-(2,2-diphenylethylene-1,1-diyl) dibenzoic acid: Energy Chemical, purity 98%;

4,4'-(1,2-diphenylethylene-1,2-diyl) dibenzoic acid: Energy Chemical, purity 91%.

Example 1

A mixed monomers of 3,3',4,4'-tetraaminobiphenyl (2.14 g), 4,4'-diphenylether dicarboxylic acid (1.9 g) and 4,4'-(2,2-diphenylethylene-1,1-diyl) dibenzoic acid (1.32 g) in a molar ratio of 1:0.735:0.315 was added to a methanesulfonic acid/phosphorus pentoxide solution (solvent 62 g) to form a reaction system, and the reaction system was reacted at 140 °C for 5 hours to obtain a reaction solution, which was introduced into water to precipitate the polymer, followed by neutralization (sodium bicarbonate) and suction filtration in sequence to obtain polymerization product PBI-1. Wherein, the weight ratio of methanesulfonic acid to phosphorus pentoxide in the methanesulfonic acid/phosphorus pentoxide solution was 12:1, the concentration of the mixed monomers in the reaction system was 8 wt%. The yield of the polymer was 99%. The hydrogen nuclear magnetic resonance spectrum of PBI-1 was measured in Test Example 6 and is shown in FIG. 1. The structure was confirmed by the hydrogen nuclear magnetic resonance spectrum.

Example 2

The method of Example 1 was followed, except that 4,4'-(2,2-diphenylethylene-1,1-diyl) dibenzoic acid was replaced by equimolar 4,4'-(1,2-diphenylethylene-1,2-diyl) dibenzoic acid. The yield of the polymer PBI-2 was 99%. The hydrogen nuclear magnetic resonance spectrum of PBI-2 was measured in Test Example 6 and is shown in FIG. 1. The structure was confirmed by the hydrogen nuclear magnetic resonance spectrum.

Example 3

The method of Example 1 was followed, except that 4,4'-diphenyletherdicarboxylic acid was replaced by equimolar 4,4'-(2,2-diphenylethylene-1,1-diyl)dibenzoic acid and the reaction time was 3 h. The yield of the polymer PBI-3 was 99%. The structure was confirmed by the hydrogen nuclear magnetic resonance spectrum.

Example 4

The method of Example 1 was followed, except that 4,4'-diphenylether dicarboxylic acid was replaced by equimolar 4,4'-(1,2-diphenylethylene-1,2-diyl) dibenzoic acid and the reaction time was 3 h. The yield of the polymer PBI-4 was 99%. The structure was confirmed by the hydrogen nuclear magnetic resonance spectrum.

Test Example 1

The polymers prepared in the above Examples were tested for thermal stability.

Testing method: the test was performed in nitrogen atmosphere using a thermogravimetric analyzer, type Q500, TA, USA. The heating rate was 10 °C/min, and the temperature range was 25-700 °C.

The thermal decomposition temperature $T_{d5\%}$ (temperature at which 5% by mass being lost) of the polymers are shown in Table 1.

TABLE 1

| | PBI-1 | PBI-2 | PBI-3 | PBI-4 |
|---|---|---|---|---|
| $T_{d5\%}$ (°C) | 490 | 530 | 510 | 490 |

Test Example 2

The free volume and the pore diameter were measured by using positron annihilation lifetime spectrum.

Testing method: the positron annihilation lifetime spectrum was tested by using DPLS3000, and the testing was conducted in atmospheric environment at room temperature and under atmospheric pressure. A 22Na positron radiation source with the activity of about $2E^{+6}$ Bq was used, the radiation source was clamped in the middle of the sample to form a sandwich structure, and 1.5 million counts were measured in total to obtain the positron annihilation life spectrum of the sample. The system had a time resolution of 190ps, a track width of 20ps, and a number of tracks of 4096. The measured positron annihilation lifetime spectrum was unscrambled by using Melt 4.0 program to obtain the positron annihilation lifetime and intensity, and to obtain the free volume and the pore diameter of the polymer.

The polymer free volume and pore diameter testing results are shown in Table 2.

TABLE 2

| | PBI-1 | PBI-2 | PBI-3 | PBI-4 |
|---|---|---|---|---|
| free volume($Å^3$) | 95 | 89 | 108 | 105 |
| pore diameter(Å) | 2.8 | 2.8 | 3.0 | 2.9 |

Test Example 3

The polybenzimidazoles were tested for isothermal adsorption properties on gases.

Testing method: the test was carried out using ASAP2020 from Micromeritics. The sample was put into a sample tube, and the sample tube was put into a degassing station for high-temperature vacuum-pumping degassing treatment. After weighing the degassed sample, the degassed sample was put into an analysis station, and the adsorption isotherms and the desorption isotherms of $N_2$, $O_2$ and $CO_2$ at room temperature were measured respectively, wherein the range of

pressure points selected of the adsorption isotherm was 10 mmHg-800 mmHg, and the range of pressure points selected of the desorption isotherm was 800 mmHg-60 mmHg.

The testing results are shown in Table 3.

TABLE 3

| | PBI-1 | PBI-2 | PBI-3 | PBI-4 |
|---|---|---|---|---|
| nitrogen ($cm^3/g$) | 0.3 | 0.3 | 0.6 | 0.6 |
| oxygen ($cm^3/g$) | 0.75 | 0.57 | 1.06 | 0.77 |
| carbon dioxide ($cm^3/g$) | 9.5 | 8.3 | 12.7 | 11.3 |
| coefficient of separation (carbon dioxide/nitrogen) | 32 | 28 | 21 | 19 |
| coefficient of separation (carbon dioxide/oxygen) | 13 | 15 | 12 | 15 |

Test Example 4

The polymers prepared in Examples 1 to 4 above were tested for mechanochromic property.

Testing method: polymer powder was placed into a solid holder (two pieces of transparent quartz plate, one of which had a recess for holding the sample) and was placed into a fluorometer (Edinburgh-steady/transient fluorescence spectrometer FLS 1000) to measure and obtain the fluorescence emission spectrum. Then, the polymer powder was ground by hand in a mortar for 30s and then placed into the solid holder, and placed again into the fluorometer to measure the fluorescence emission spectrum.

The shifts in strongest fluorescence emission peaks before and after grinding of polymers are shown in Table 4.

TABLE 4

| | PBI-1 | PBI-2 | PBI-3 | PBI-4 |
|---|---|---|---|---|
| shift (nm) of fluorescence emission peak of PBI after being subjected to external force | 10* | 20# | 15# | 24# |
| Note: "#" indicates a blue shift of the emission peak; "*" indicates a red shift of the emission peak. | | | | |

Test Example 5

The polymers prepared in Examples 1-4 above were tested for intrinsic viscosity.

Testing method: PBI was dissolved in concentrated sulfuric acid to prepare a sulfuric acid solution with the concentration of 0.6 g $dL^{-1}$. The sulfuric acid solution was added into a Ubbelohde viscometer, the viscometer was placed into a constant-temperature water bath at 25 °C for stabilization for 30min, and the outflow time of the solution was measured, measuring each sample for three times and the time difference being not more than 1s, wherein the average value was obtained and recorded as $t_1$, and the outflow time of concentrated sulfuric acid was recorded as $t_0$. The intrinsic viscosity [η] of PBI was calculated according to the following equations, the specific viscosity $\eta_{SP} = \frac{t_1 - t_0}{t_0}$ and the intrinsic viscosity $[\eta] = \frac{\eta_{SP} + 3\ln(1+\eta_{SP})}{4C}$, wherein C is the concentration of the PBI sulfuric acid solution.

The testing results are shown in Table 5.

TABLE 5

| | PBI-1 | PBI-2 | PBI-3 | PBI-4 |
|---|---|---|---|---|
| intrinsic viscosity (dL $g^{-1}$) | 2.18 | 1.08 | 1.75 | 2.01 |

Test Example 6

The polymers of Example 1 and Example 2 were tested for hydrogen nuclear magnetic resonance spectra. The hydrogen nuclear magnetic resonance spectrum testing method: Agilent 400-MR DD2 nuclear magnetic resonance

spectrometer with the frequency of 400 MHz being used, tetramethylsilane (TMS) as internal standard substance, DMSO-$d_6$ as solvent and the testing temperature being 40 °C.

The hydrogen nuclear magnetic resonance spectra of the polymerization products of Example 1 and Example 2 are shown in Fig. 1, wherein the proton signal at chemical shift 13.0ppm in the spectra can be assigned to the characteristic peak of active hydrogen in PBI structural unit, the proton signals at 7.30ppm and 8.28ppm can be assigned to the characteristic peaks of corresponding structural unit of 4,4'-diphenyl ether dicarboxylic acid monomer after polymerization, and the proton signals at 7.08-7.24ppm and 7.55-8.02ppm can be assigned respectively to the characteristic peaks of corresponding structural units of tetramine monomer and tetraphenyl ethylene diacid monomer after polymerization . The results of spectra analysis show that the polymers PBI-1 and PBI-2 were successfully obtained.

The results of hydrogen nuclear magnetic resonance spectra show that the polymers PBI-1 to PBI-4 were successfully obtained.

Comparative Example 1

The data reported in the following document was used:
Facile preparation of porous polybenzimidazole networks and adsorption behavior of $CO_2$ gas, organic and water vapors, Hao Yu et al., Polym. Chem., 2013, 4, 961-968 (DOI:10.1039/c2py20908j)

The experiments in this document used Autosorb iQ-MP test, wherein the adsorption isotherms of the gases were measured at room temperature respectively, with an upper pressure limit of 1 bar. The testing results are shown in Table 6.

TABLE 6

| | PBI-1' | PBI-2' |
|---|---|---|
| coefficient of separation (carbon dioxide/nitrogen) | 12.3 | 9.3 |

Comparative Example 2

The data reported in the following document was used:
Influence of polybenzimidazole main chain structure on $H_2/CO_2$ separation at elevated temperatures, Xin Li et al, Journal of Membrane Science, Volume 461, 1 July 2014, Pages 59-68 (DOI:10.1016/j.memsci.2014.03.008)

The experiments in this document used Kalrez™ test at pressure of 10-50psi and at temperature of 30-250 °C. The testing results are shown in Table 7.

TABLE 7

| | 6F-PBI | BTBP-PBI | phenyl indan-PBI | PFCB-PBI | m-PBI |
|---|---|---|---|---|---|
| coefficient of separation (carbon dioxide/nitrogen) | 3.6 | 3.3 | 4 | 3.6 | 4.3 |

The preferred embodiments of the present invention have been described above in detail, but the present invention is not limited thereto. Within the scope of the technical concept of the invention, many simple modifications can be made to the technical solutions of the invention, including combining various technical features in any other suitable ways, and those simple modifications and combinations should also be regarded as the disclosure of the invention, and all fall within the protection scope of the invention.

## Claims

1. A polybenzimidazole, **characterized in that** the polybenzimidazole comprises structural unit A, and the structural unit A has a structure represented by formula (1);

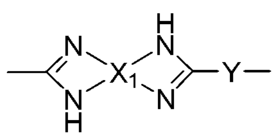

formula (1)

wherein, the $X_1$ group is a linking group provided by an aromatic hydrocarbon compound containing 1-10 benzene rings; and the Y group is a tetraphenylethylene-based linking group; wherein, the $X_1$ group and the Y group are each independently not substituted or substituted by at least one substituent selected from the group consisting of hydroxyl, amino, cyano, halogen, $C_1$-$C_4$ alkyl, and $C_1$-$C_4$ haloalkyl.

2. The polybenzimidazole according to claim 1, wherein the polybenzimidazole comprises structural unit A and structural unit C, comprises structural unit A and structural unit D, or comprises structural unit A, structural unit B, structural unit C and structural unit D;
wherein the structural unit A has a structure represented by formula (1), the structural unit B has a structure represented by formula (2), the structural unit C has a structure represented by formula (1-1), and the structural unit D has a structure represented by formula (2-2):

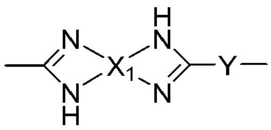 formula (1) 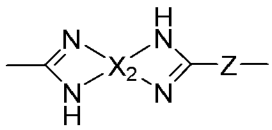 formula (2)

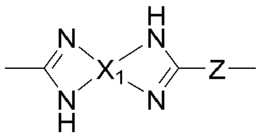 formula (1-1) 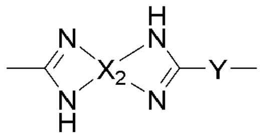 formula (2-2)

wherein, the $X_1$ and $X_2$ groups are each independently a linking group provided by an aromatic hydrocarbon compound containing 1-10 benzene rings; the Y group is a tetraphenylethylene-based linking group; and the Z group is a linking group provided by at least one compound selected from the group consisting of an aromatic hydrocarbon containing 1-10 benzene rings, a heterocyclic aromatic hydrocarbon containing 1-10 heterocyclic rings, an aromatic hydrocarbon containing 1-3 heterocyclic rings and 1-8 benzene rings, a $C_4$-$C_{10}$ cycloalkane, a $C_1$-$C_8$ paraffinic hydrocarbon and a $C_2$-$C_8$ olefin; wherein, the $X_1$, $X_2$, Y and Z groups are each independently not substituted or substituted by at least one substituent selected from the group consisting of hydroxyl, amino, cyano, halogen, $C_1$-$C_4$ alkyl, and $C_1$-$C_4$ haloalkyl.

3. The polybenzimidazole according to claim 1, wherein the polybenzimidazole comprises structural unit A and optionally structural unit B, the structural unit A having a structure represented by formula (1) and the structural unit B having a structure represented by formula (2);

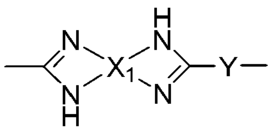 formula (1) 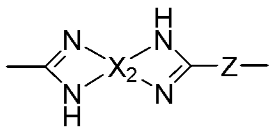 formula (2)

wherein, the $X_1$ and $X_2$ groups are each independently a linking group provided by an aromatic hydrocarbon compound containing 1-10 benzene rings; the Y group is a tetraphenylethylene-based linking group; and the Z group is a linking group provided by at least one compound selected from the group consisting of an aromatic hydrocarbon containing 1-10 benzene rings, a heterocyclic aromatic hydrocarbon containing 1-10 heterocyclic rings, an aromatic hydrocarbon containing 1-3 heterocyclic rings and 1-8 benzene rings, a $C_4$-$C_{10}$ cycloalkane, a $C_1$-$C_8$ paraffinic hydrocarbon and a $C_2$-$C_8$ olefin, and Z is different from Y when the structural unit B exists; wherein, the $X_1$, $X_2$, Y and Z groups are each independently not substituted or substituted by at least one substituent selected from the group consisting of hydroxyl, amino, cyano, halogen, $C_1$-$C_4$ alkyl, and $C_1$-$C_4$ haloalkyl.

4. The polybenzimidazole according to any one of claims 1 to 3, wherein the $X_1$ and $X_2$ groups are each independently a linking group provided by at least one compound selected from the group consisting of benzene, biphenyl, terphenyl, a bridged-benzenes, an iptycene, and a fused ring aromatic hydrocarbon containing 1 to 10 benzene rings; preferably, the $X_1$ and $X_2$ groups are each independently a linking group provided by at least one compound selected from the group consisting of benzene, biphenyl, a bridged-benzenes, an iptycene and naphthalene; more preferably, the bridged-benzenes has a structure represented by formula (3), formula (4) or formula (5), wherein $R_1$, $R_3$, $R_4$ are each

independently selected from the group consisting of oxygen atom, sulfur atom, carbonyl, sulfonyl, methylene, and halogen-substituted methylene; and $R_2$ is selected from the group consisting of heteroatom-containing aromatic rings;

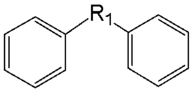 formula (3) 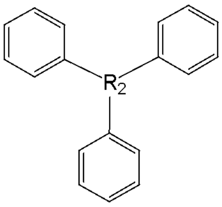 formula (4) 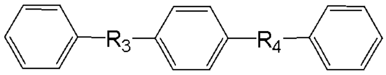 formula (5).

**5.** The polybenzimidazole according to any one of claims 1-4, wherein the $X_1$ and $X_2$ groups are each independently selected from the following linking groups:

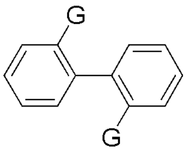 、 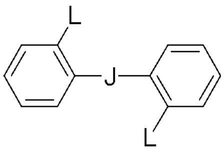 、 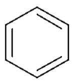 、 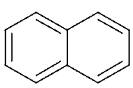 、

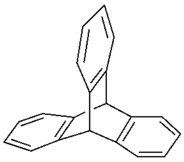 、 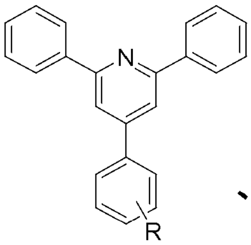 、 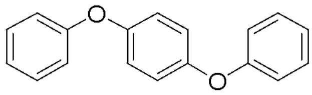 ;

wherein G is selected from hydrogen atom, methyl and trifluoromethoxy; J is selected from oxygen atom, sulfur atom, carbonyl, sulfonyl, methylene and methyl- or trifluoromethyl-substituted methylene; L is selected from bromine atom, phenyl and trifluoromethyl-substituted phenyl; and R is selected from hydrogen atom, carboxyl, hydroxyl and trifluoromethyl.

**6.** The polybenzimidazole according to any one of claims 2 to 5, wherein the Z group is a linking group provided by at least one compound selected from the group consisting of benzene, a 5-6 membered nitrogen-containing heterocyclic compound, an aromatic hydrocarbon compound containing 2-7 benzene rings, an aromatic hydrocarbon compound containing 1-2 nitrogen-containing heterocyclic rings and 1-4 benzene rings, a $C_4$-$C_{10}$ cycloalkane, a $C_1$-$C_8$ paraffinic hydrocarbon and a $C_2$-$C_8$ olefin;
preferably, the Z group is a linking group selected from:

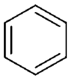 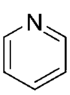 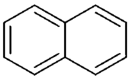 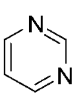 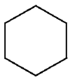 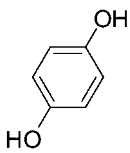 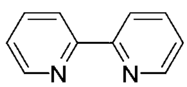 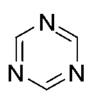

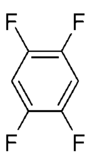 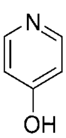 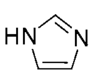 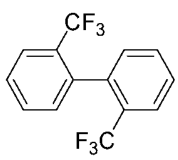 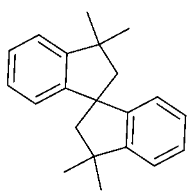 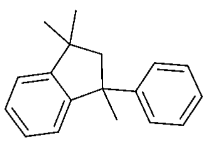

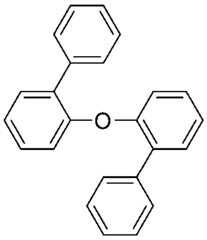
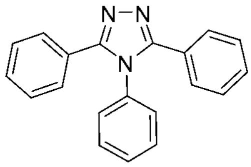
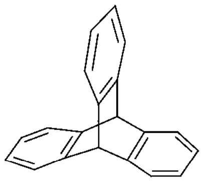
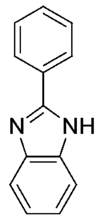
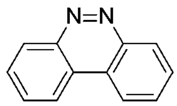
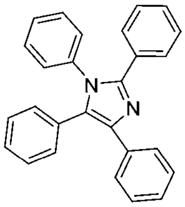
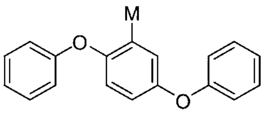
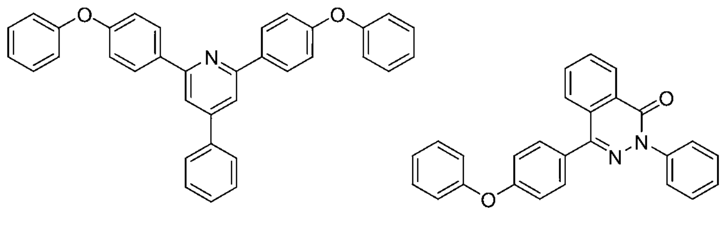
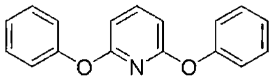
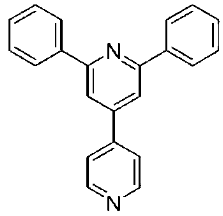
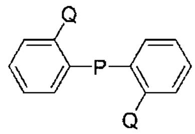
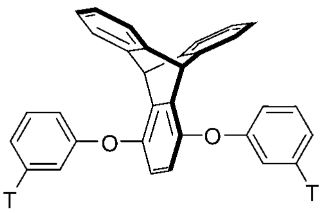
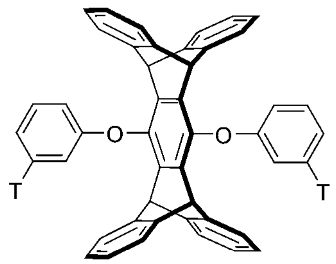

,

$C_1$-$C_8$ paraffinic hydrocarbon group;
wherein M is selected from the group consisting of hydrogen atom, cyano, methyl, phenyl, and methyl- or trifluoromethyl-substituted phenyl; P is selected from the group consisting of oxygen atom, sulfur atom, carbonyl, sulfonyl, cyclobutyl, halogen-substituted cyclobutyl, methylene and methyl- or trifluoromethyl-substituted methylene; Q is selected from the group consisting of bromine atom, phenyl and trifluoromethyl-substituted phenyl; and T is selected from the group consisting of hydrogen atom and methyl.

**7.** The polybenzimidazole according to any one of claims 1-6, wherein the Y group is derived from at least one of the following compounds:

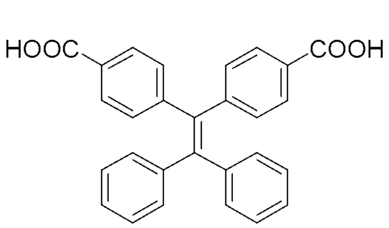

and

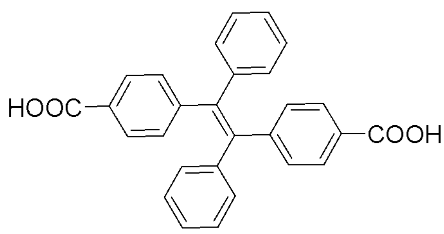

.

**8.** The polybenzimidazole according to any one of claims 1-7, wherein

the polybenzimidazole has an intrinsic viscosity of 0.45-3.0 dL $g^{-1}$; and/or

the molar content ratio of $X_2$ group to $X_1$ group in the polybenzimidazole is 0-9999, preferably 0-99; and/or the molar content ratio of Z group to Y group in the polybenzimidazole is 0-9999, preferably 0-99.

**9.** The polybenzimidazole according to any one of claims 1 to 8, wherein the polybenzimidazole has a free volume of 80-120Å$^3$, preferably 85-110Å$^3$; and the polybenzimidazole has a pore diameter of 2.5-3.5Å, preferably 2.6-3.2Å.

**10.** The polybenzimidazole according to any one of claims 1 to 9, wherein the polybenzimidazole has mechanochromic property.

**11.** A method for the preparation of the polybenzimidazole according to any one of claims 1 to 10, wherein the method comprises reacting at least one compound having the structure represented by formula (6) with HOOC-Y-COOH and optionally HOOC-Z-COOH;

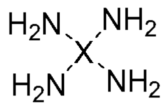 formula (6)

wherein the X group in formula (6) is the same as the $X_1$ and/or $X_2$ groups as defined in any one of claims 1-10, and the Y and Z groups in HOOC-Y-COOH and optionally HOOC-Z-COOH are respectively the same as the Y and Z groups as defined in any one of claims 1-10.

**12.** The method according to claim 11, wherein the reaction is carried out in a solvent, preferably the solvent is polyphosphoric acid or a mixed solution of methanesulfonic acid/phosphorus pentoxide, further preferably a mixed solution of methanesulfonic acid/phosphorus pentoxide and the mass of phosphorus pentoxide is 2-10% of the mass of methanesulfonic acid.

**13.** The method according to claim 11 or 12, wherein the ratio of the molar amount of the compound having the structure represented by formula (6) to the total molar amount of HOOC-Y-COOH and optionally HOOC-Z-COOH is 1:0.6-2, preferably 1:0.8-1.2.

**14.** A method for separating $CO_2$ gas by adsorption, wherein the polybenzimidazole according to any one of claims 1 to 10 is used as adsorbent.

**15.** The method according to claim 14, wherein the $CO_2$ gas is separated by adsorption from a gas selected from the group consisting of $N_2$, $O_2$, or a mixture thereof.

**16.** Use of the polybenzimidazole according to any one of claims 1 to 10 as adsorbent for gas adsorption separation.

**17.** The use according to claim 16, wherein the polybenzimidazole is used as adsorbent for separating $CO_2$ gas from a gas selected from the group consisting of $N_2$, $O_2$ or a mixture thereof.

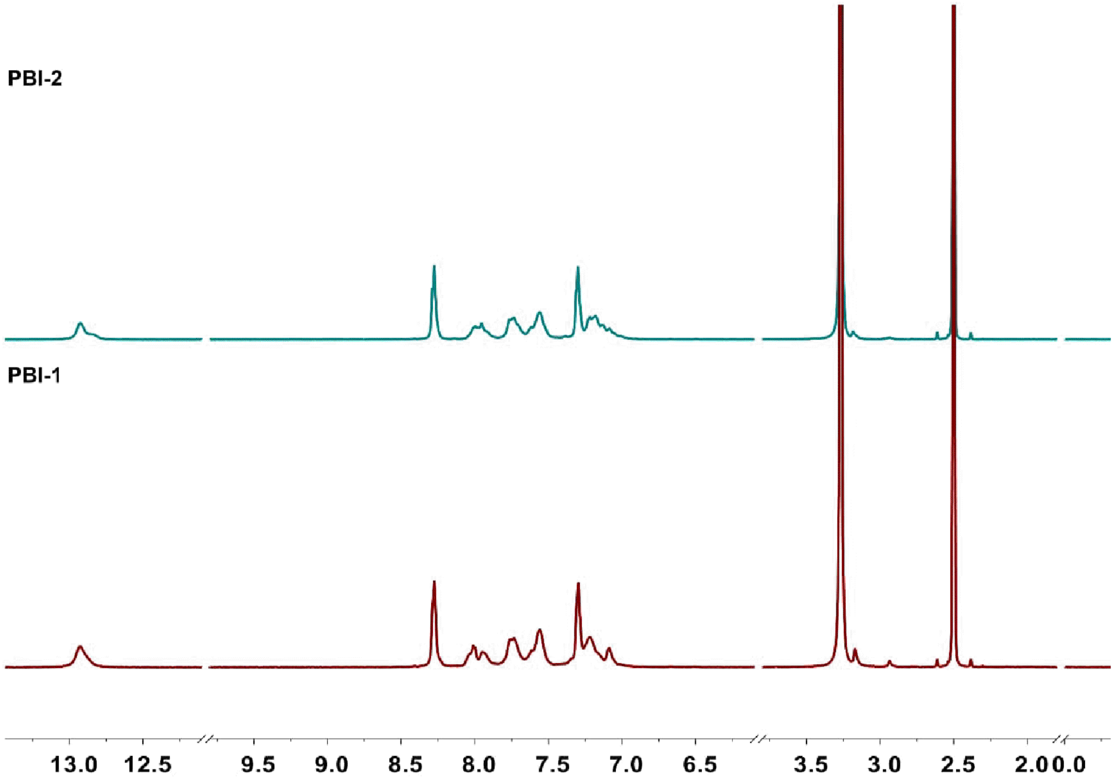
PBI-2
PBI-1
13.0
12.5
9.5
9.0
8.5
8.0
7.5
7.0
6.5
3.5
3.0
2.5
2.00.0

Fig. 1

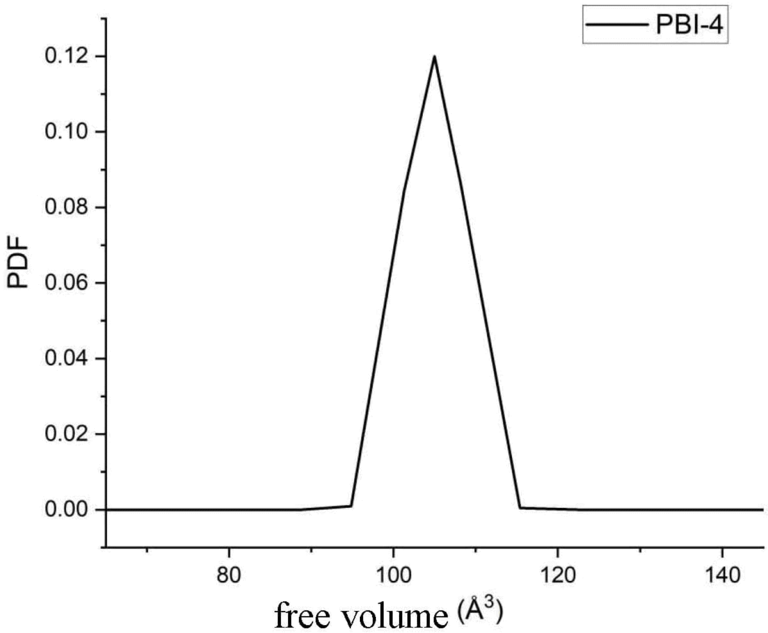
PBI-4
PDF
0.12
0.10
0.08
0.06
0.04
0.02
0.00
80
100
120
140
free volume (Å3)

Fig. 2

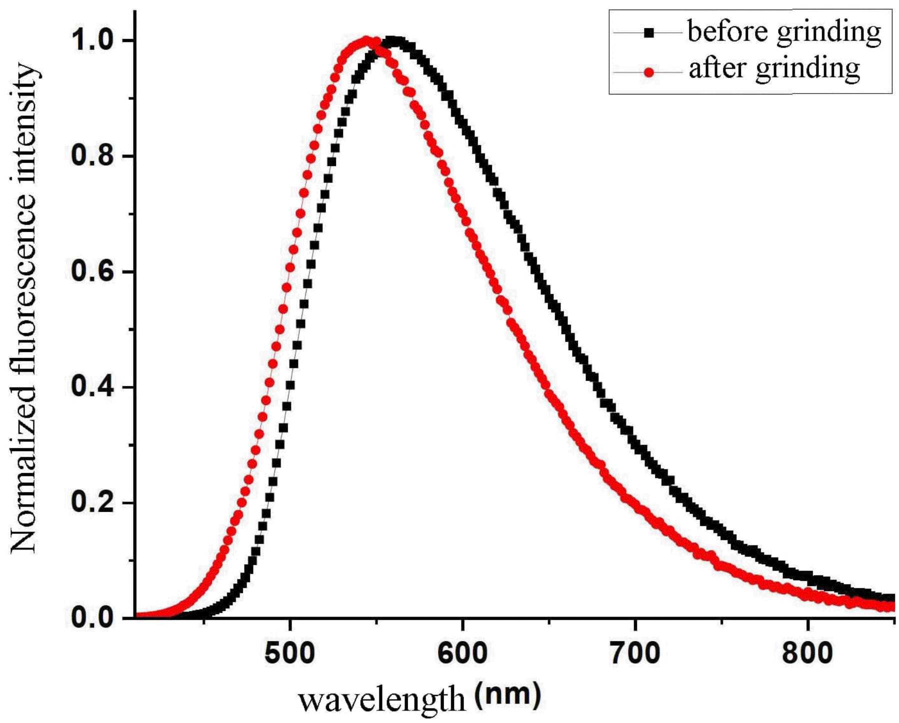
before grinding
after grinding
Normalized fluorescence intensity
1.0
0.8
0.6
0.4
0.2
0.0
500
600
700
800
wavelength (nm)

Fig. 3

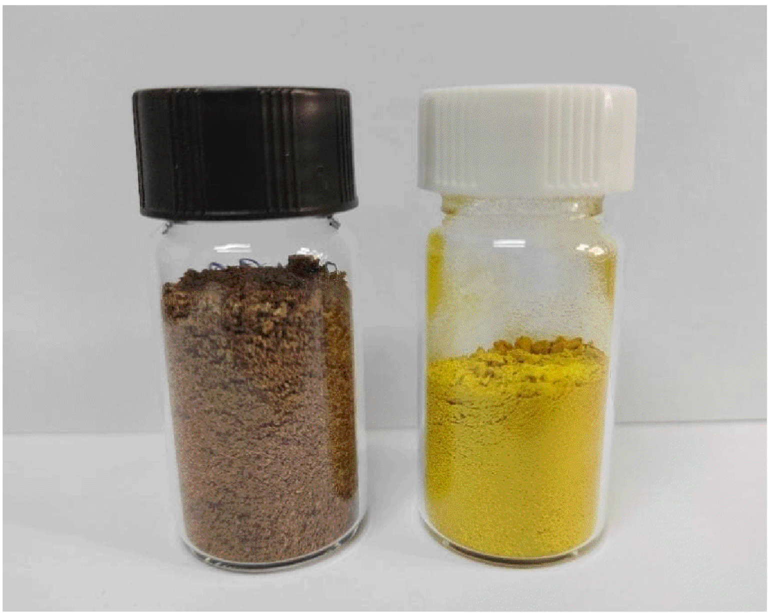

Fig. 4

**INTERNATIONAL SEARCH REPORT**

International application No.
**PCT/CN2024/109412**

**A. CLASSIFICATION OF SUBJECT MATTER**

C08G73/18(2006.01)i; C07D235/04(2006.01)i

According to International Patent Classification (IPC) or to both national classification and IPC

**B. FIELDS SEARCHED**

Minimum documentation searched (classification system followed by classification symbols)

C08G73/-; C07D235/-

Documentation searched other than minimum documentation to the extent that such documents are included in the fields searched

Electronic data base consulted during the international search (name of data base and, where practicable, search terms used)

CNTXT, CNKI, WPABS, STN: 苯并咪唑, 二苯基乙烯, 二苯甲酸, 发光, 聚苯并咪唑, 力致变色, 热, 四苯乙烯, 荧光, PBI, polybenzimidazole, tetraphenylethylene, TPE, benzimidazole

**C. DOCUMENTS CONSIDERED TO BE RELEVANT**

| Category* | Citation of document, with indication, where appropriate, of the relevant passages | Relevant to claim No. |
|---|---|---|
| A | CN 103055723 A (LIAONING NORMAL UNIVERSITY) 24 April 2013 (2013-04-24) claim 1 | 1-17 |
| A | CN 107188853 A (CHINA UNIVERSITY OF MINING AND TECHNOLOGY) 22 September 2017 (2017-09-22) claim 1 | 1-17 |
| A | CN 113773491 A (SOUTH CHINA NORMAL UNIVERSITY) 10 December 2021 (2021-12-10) claim 1 | 1-17 |
| A | US 2010292356 A1 (IUCF-HYU (INDUSTRY-UNIVERSITY COOPERATION FOUNDATION HANYANG UNIVERSITY)) 18 November 2010 (2010-11-18) description, paragraphs 13-15 | 1-17 |
| A | CN 112210057 A (SHANGHAI NORMAL UNIVERSITY et al.) 12 January 2021 (2021-01-12) claims 1-3 | 1-17 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 October 2024** | **30 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/109412**

| Patent document cited in search report | | | Publication date (day/month/year) | Patent family member(s) | | | Publication date (day/month/year) |
|---|---|---|---|---|---|---|---|
| CN | 103055723 | A | 24 April 2013 | None | | | |
| CN | 107188853 | A | 22 September 2017 | None | | | |
| CN | 113773491 | A | 10 December 2021 | None | | | |
| US | 2010292356 | A1 | 18 November 2010 | US | 8642722 | B2 | 04 February 2014 |
| CN | 112210057 | A | 12 January 2021 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HAO YU et al.** *Polym. Chem.,*, 2013, vol. 4, 961-968
- **XIN LI et al.** *Journal of Membrane Science*, 01 July 2014, vol. 461, 59-68